# EUROPEAN PATENT SPECIFICATION

(11) **EP 1 652 480 B1**
(45) Date of publication and mention of the grant of the patent: **14.01.2009**
(21) Application number: 04766894.2
(22) Date of filing: 30.07.2004
(51) Int. Cl.: A61B 17/02

(54) **SELF-RETAINING SURGICAL RETRACTOR**
SELBSTHALTENDER CHIRURGISCHER WUNDHAKEN
ECARTEUR CHIRURGICAL AUTOSTATIQUE

(30) Priority: 05.08.2003 ES 200301876
(43) Date of publication of application: 03.05.2006
(73) Proprietor: Laucirica Gari, Oscar, 08980 Sant Feliu de Llobregat (ES)
(72) Inventor: Laucirica Gari, Oscar, 08980 Sant Feliu de Llobregat (ES)
(74) Representative: Aragones Forner, Rafael Angel
(86) International application number: PCT/ES2004/000355
(87) International publication number: WO 2005/013833

(56) References cited:
- ES-A1- 8 103 963
- ES-A1- 8 403 020
- ES-T3- 2 121 159
- ES-U- 1 050 254
- US-A- 4 010 741
- US-A- 5 520 610
- US-B1- 6 241 659
- US-B1- 6 431 025

## Description

### OBJECT OF THE INVENTION

The present invention has as its object an autostatic surgical separator being apt to be applied in the surgical operations being preferentially performed on the abdomen, thorax, back, perineum and lumbar region of a patient.

### BACKGROUND OF THE INVENTION

In order to perform surgical operations on patients a surgical incision is normally carried out in the first place thus opening the operative field to the intra-abdominal or corresponding contents of the patient in order to thus thereupon be in a position to perform the corresponding surgical operation.

Autostatic or self-retaining surgical separators are used in order to *en bloc* fix and separate the lips of the surgical incision to thus allow performing the operation.

There are several patent documents disclosing the corresponding separator assemblies as well as several of their components and accessories, and from among said documents can be cited, among others, the patents US 4,254,763 and US Appl. 2002-0026101 to Codman & Shurtleff Inc. relating to the separator or retractor assembly; US 6,241,659 (corresponding EP Application no. 1 090 589) and US 5,375,481 to Codman & Shurtleff Inc. relating to the fixing mechanism or device being provided to fix the valvae arms; US 5,520,608 to Johnson & Johnson Professional relating to a valva; and a number of components and accessories being disclosed in patents US 5,365,921; US 5,356,100; US 5,320,314; US 5,320,444 and US 4,467,791 also to Codman and having been applied for by several inventors.

The precited Codman & Shurtleff patents have mechanical limitations as regards the slotting of the valvae pulling arms.

Said drawbacks have been partially eliminated by the Spanish Utility Model no. 200102527 (Publ. no. ES 1 050 254 U) of this same applicant notably improving the fixing device being provided to fix the valvae arms as well as the very arms and the lighting of the patient's region.

Said utility model essentially comprises the field opening ring, pulling valvae of several dimensions and configurations being provided with pulling arms of square cross-section having their upper face slotted, fixing devices being each provided with a rectangular channel in correspondence with the valvae pulling arms thus allowing to fit and lock/unlock said arms in order to thus fix said valvae in position, and orientable lighting means.

The precited, already granted Spanish utility model has nevertheless some limitations as regards its operation and use, and it has a complex makeup as regards the valvae arms and the corresponding fixing devices.

### SUMMARY OF THE INVENTION

The above-mentioned and other drawbacks and limitations regarding the makeup of the previous utility model and of the different patents having been cited above, especially the Codman patents, have now been totally obviated by means of the surgical separator of the present invention.

The separator being the object of the present invention has notable differences as compared with the precited documents, and especially with the Codman patents, and from among its main features it can be pointed out that the valvae arms are in this case not slotted, the separator of the invention hence allowing to incorporate the valva in any position (this not being possible in Codman) and to "micrometrically" retract the valva (there are no limitations as defined by a slotting of the arm), the separator offers a better hygiene and a better comfort as regards the positions being occupied by the different members of the surgical team around the operative field, the external fixation device is much more versatile, more reduced and less heavy than in Codman and is apt to be much more rapidly fitted, and the lighting device being provided with a cold light provides an orientable lamp (not being provided by Codman). Codman also has in the slot of the base plate of the fixing device a curved projection such that when the fixing device is fitted to the ring said curved projection can only be fitted into the valley of the corresponding notch of the ring, whereas the fixing device of the instant invention is at the bottom of said slot advantageously devoid of such projection, so that the fixing device of the invention can be fitted at the valley or at the peaks of the ring notches and is thus better adaptable to the ring for its fixation, and the valva can also be better adapted to the tissue planes.

The valvae arms of the Codman patents have a quadrangular cross-section and can hence only fit in one position into the longitudinal channel having a corresponding cross-section in the respective fixing device, whereas in the present invention the valvae arms are made up with any cross-section, this latter being preferentially circular or oval-shaped as the case might be, in the preferential embodiment being illustrated in the drawings the valvae arms of the invention hence having a round cross-section in correspondence with the same cross-section of the longitudinal channel of the fixing device, this allowing the valvae arms of the invention to be fitted into the fixing device channel in any angular position.

The instant invention is defined by the appended set of claims.

The field opening ring having an open or closed configuration is essentially planar and is exteriorly provided with a succession of arcuate notches.

The pulling arms have a smooth surface and any cross-section, this latter preferentially being circular or oval-shaped in correspondence with the longitudinal channel of the fixing device and the aperture of the apertured dog of the fixing device, said aperture and arm having the same cross-section with a slight play. Said pulling arms are each made up as a rigid, one-piece member, or else they can be articulated at an intermediary position thus occupying less space.

The fixing devices being provided to fix the valvae arms to the field opening ring are of a fixed or tillable type with respect to said ring. The tillable fixing device comprises a swivel pin being located at the front face between the central body and the slotted base plate and allowing to carry out an angular motion between both parts, said angular motion being lockable at different angular values by means of the incorporation of a tubular structure having an arcuate concave front face and establishing contact with a projection of the back face of the base plate of the fixing device, a special spring being provided which maintains the arcuate, tubular structure with the locking notches in position against the base plate body thereby maintaining the inclination of the fixing device main body and hence of the corresponding valva arm. The fixing device of the fixed type in its turn consists in a one-piece member integrally comprising the central body and the slotted base plate.

As for the multiarticulated supporting arm, a second tube is apt to slide inside each tube, said second tube ending in a 45° chamfering at the level of the central locking thread, in such a way that in said locking arrangement the locking thread articulates two bodies having a tapered section such that when threadingly tightening the assembly the taperingly driving bodies are brought nearer to one another thus simultaneously pushing the inner tubes of each tube against the ball joint arrangement being provided at each end, the whole assembly making up the articulated arm being thus totally locked, a three-dimensional movability being thus provided for the ball joint arrangement in combination with the thread.

The orientable cold light has a mechanism comprising two articulated tubular structures, said mechanism being similar to that of the multiarticulated arm having been described above. Said light comprises a lamp being made up by the precited multiarticulated support, a support being apt to be fitted to the field opening ring, a cup being provided with an optical lens at its end, the cup being threadingly engaged with the multiarm support and at the free end being provided with a thread for the fixation of the optical lens, said cup at the opposite end having a thread allowing to fit the corresponding optical fibre tube.

These and other features are being highlighted in the accompanying figures illustrating an exemplary embodiment of the invention having a merely illustrative character and in no way limiting the scope of the present invention.

### DESCRIPTION OF THE DRAWINGS

In said drawings:
Fig. 1 is a diagrammed, perspective view of the autostatic surgical separator.
Fig. 2 is a perspective view of the separator of the invention in use position on the patient.
Fig. 3 is a perspective view from the back showing the fixing device of the tiltable type being fitted to the ring.
Fig. 4 is an elevational view of the device of Fig. 3 having been in this case isolated.
Fig. 4A is a diagrammed elevation showing the device of Figs. 3 and 4.
Fig. 5 is a perspective view from the back of the fixing device of the fixed type showing the valva arm being articulated at an intermediary position.
Fig. 6 is a diagrammed plan-view showing the set of components and accessories of the separator of the invention.
Fig. 7 is a plan-view of the cold light with the associated multiarticulated arm.
Fig. 8 is an exploded view of the multiarticulated supporting arm being provided to support the ring.
Fig. 9 is a view being similar to the Fig. 8 but in this case showing the clamping handle in its assembled state.
Fig. 10 is a diagrammed, fragmentary elevation showing the orientable cold light.
Fig. 11 is a diagrammed, essentially sectional, fragmentary view showing the gripping clamp being provided to allow the multiarticulated arm to grip the ring.
Fig. 12 is a diagrammed, perspective view of the gripper being provided to grip the supporting rod being provided to support the multiarticulated support, said gripper being provided with a clamp being apt to securely grip the support of the corresponding operating table.

### DETAILED DESCRIPTION OF A PREFERRED EMBODIMENT

According to the drawings the present invention consists in an autostatic surgical separator being generally referenced at -1- for automatically and *en bloc* holding and separating the skin and the tissue planes around the lips of the surgical incision, said autostatic surgical separator being of the type comprising field opening means being provided for opening the field within said lips, valvae means being provided for pulling said lips and fitted into respective fixing devices being applied along the opening means, supporting means being provided for supporting the opening means, and lighting means being provided for lighting the field area.

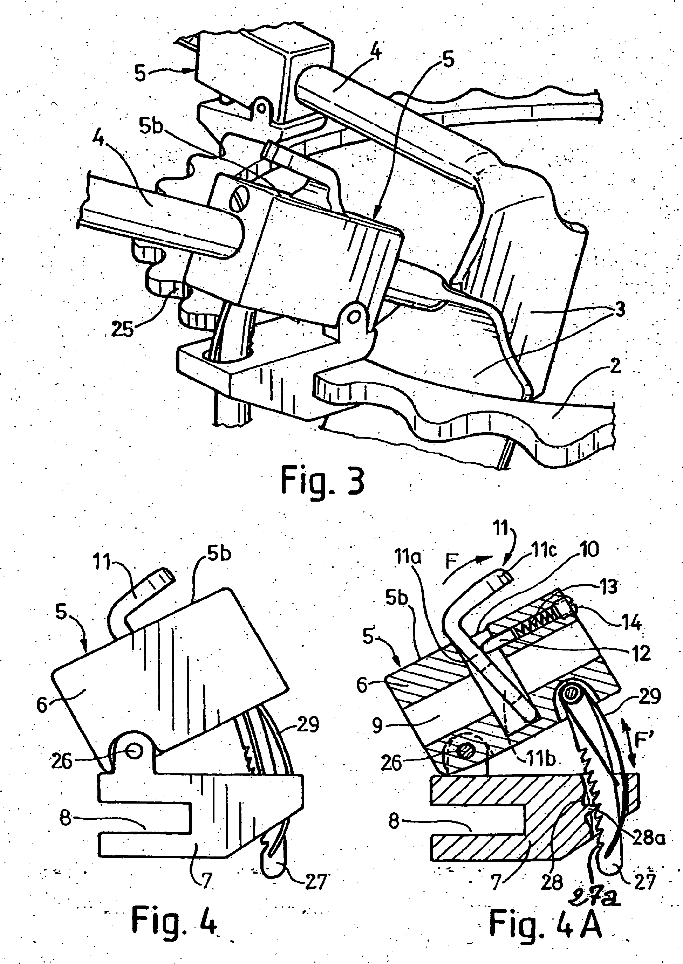

The present invention has as its object a separator -1- comprising in combination:
- field opening means being made up by way of ring - 2- having a closed and/or open configuration and any shape and size;
- pulling valvae means -3- of different configurations being integral with the corresponding pulling arm -4- having a smooth surface and any cross-section, this latter being preferentially circular or oval-shaped, said pulling arm having been illustrated in the drawings with a circular surface, this latter being the preferred one;
- fixing devices -5- allowing to fix respective pulling valvae -3- being fitted into them by means of the corresponding arms -4- to the field opening ring, said fixing devices comprising a central body -6- being fitted to a base plate -7- being slotted at -8- and by means of which the fixing device is fixed to the field opening ring -2-, said slot -8- being devoid of a projection of any type and thus having a flat bottom allowing the fixing device -5- to be fixed at the valleys or at the peaks of the notches of said ring -2-, said central body -6- of the fixing device -5- having a longitudinal channel -9- whose cross-section is in correspondence with that of the pulling arm -4- of the respective valva -3-(being illustrated in the drawings with a circular section, this being the preferred shape), said central body as well upperly having a transversal slit -10- allowing to fit into it an apertured dog -11- through which the arm -4- being provided for pulling the valva - 3- is apt to slide, said upper apertured dog -11- being made up by a plate -11a- being provided with an aperture -11b- and an upper appendage -11c- acting as the very dog, said aperture -11b- having a contour that with a slight play is essentially in correspondence with the circular cross-section (or with the cross-section of any other shape) of the valva arm -4-, said dog -11- being permanently biased in a forward direction by a stub -12- being in its turn biased by resilient means being made up by a spring -13- and maintained in position by means of a screw -14-, said arrangement allowing the arm -4- being provided for pulling the valva -3- to slide through the aperture of the plate of the upper apertured dog -11- when sliding through the longitudinal channel -9- having, been also illustrated with a circular cross-section (which is the preferential cross-section), said aperture -11b- of the plate -11a- of the apertured dog -11- locking the arm -4- being provided for pulling the valva -3- when said arm attempts to move in the same direction and in the opposite sense, the pulling arm -4- thus being locked inside the fixing device -5-, the locking pressure being exerted by the apertured dog -11- on the pulling arm -4- becoming higher when there is an increase of the pressure being exerted on the valva -3-, said locking being released when the user proceeds to digitally actuate said apertured dog -11- in a backward direction as per arrow -F- (Fig. 4A) thus allowing the corresponding arm -4- being provided for pulling the corresponding valva to be easily slid.

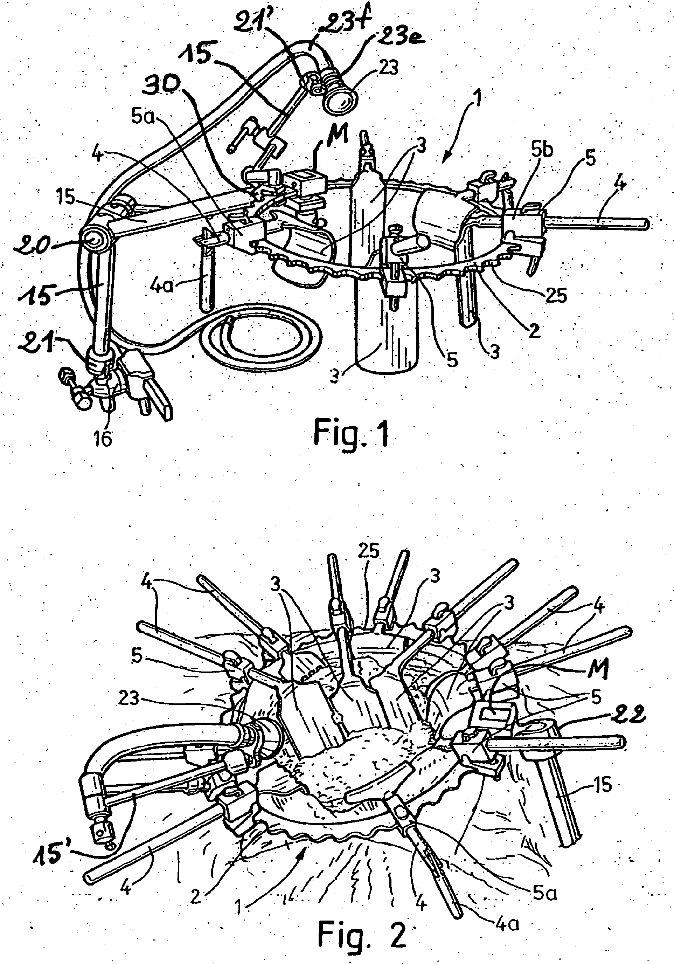

According to the invention the fixing device -5- can be of the fixed -5a- (Figs. 1, 5 and 6) or of the tiltable -5b- type (Figs. 1, 3, 4, 4A and 6) with respect to the field opening ring -2-.

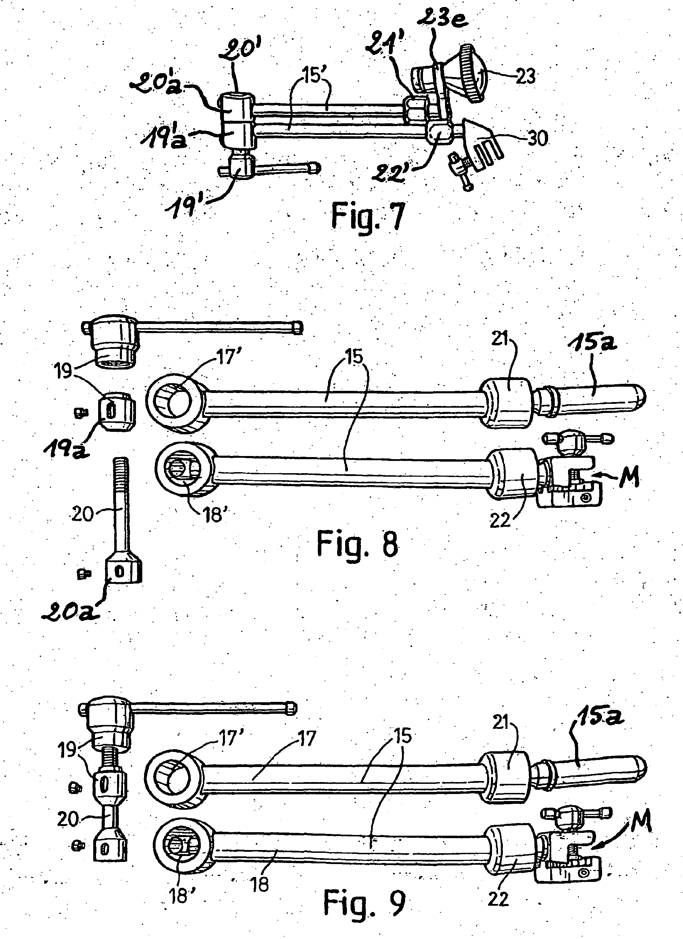

The separator -1- comprises a multiarticulated supporting arm -15- (Figs. 1, 2, 6, 8 and 9) being provided for supporting the field opening ring -2- and having a mechanism allowing to quickly and easily fix one end of said arm to the corresponding support -16- (Figs. 1, 6 and 12) and the opposite end to the field opening ring -2- and comprising two tubular structures -17-, -18- being articulated at one -19- of their ends (Figs. 8 and 9) by means of a central locking thread -20- and at the other end of each tube having a ball joint arrangement-21-, -22- at one side articulating the rod being incorporated into the lateral support being provided for supporting the arm, said ball joint arrangement at the other side articulating the gripping clamp being provided to fix the field opening ring.

The separator -1- comprises an orientable cold light -23- being provided at the end of a multiarticulated arm -24- being similar to the arm -15- of the above-mentioned support and having a mechanism allowing to quickly and easily fix one end of said arm to the field opening ring, said orientable light being provided at the opposite end.

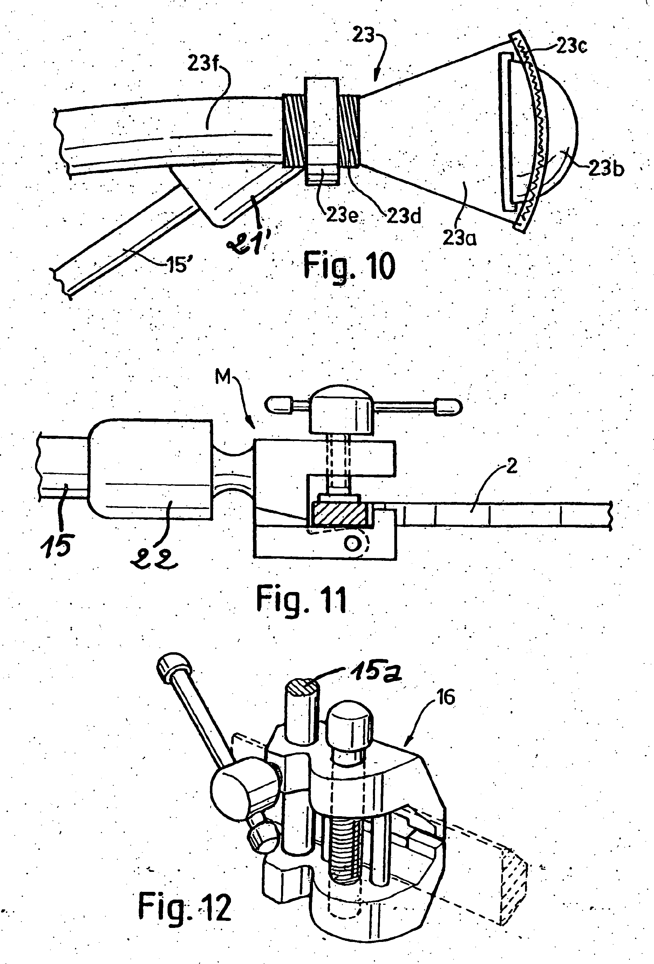

Said orientable cold light -23- comprises a hollow cup -23a- to one of whose ends an optical lens -23b- is fitted and for such a purpose is fixed by means of a nut -23c-, said cup being at the opposite end provided with a thread -23d- threadingly engaging the, end -23e- of the support -15'-, this latter at its opposite end being fixed by means of the corresponding clamp to the ring -2-, said thread -23d- threadingly engaging the corresponding optical fibre cable -23f- (Fig. 10).

The field opening ring -2- being of the open or of the closed type is essentially planar and is exteriorly provided with a succession of arcuate notches -25-.

The pulling arms -4- have a smooth surface and any cross-section, this latter being preferentially circular (such as illustrated in the drawings) and/or oval-shaped, the aperture -11b- of the plate -11a- of the apertured dog -11- of the fixing device -5- in correspondence with said cross-sectional shape having the same cross-section with a slight play. Said pulling arms -4- are made up by a rigid one-piece member, or else they can be articulated -4a- at an intermediary position thus occupying less space.

The arms -4-, the longitudinal channel -9- of the fixing device -5- (-5a-, -5b-) and the aperture -11b- of the plate -11a- of the apertured dog -11- have been illustrated in the drawings with a circular cross-section or contour, this being the preferred configuration allowing said arms -4- to be fitted into the fixing device in any angular position.

The fixing devices -5- being provided for fixing the arms -4- of the valvae -3- to the field opening ring -2-are of the fixed -5a- and/or of the tiltable -5b- type with respect to said ring. The tiltable fixing device-5b- comprises a swivel pin -26- being located at the front face between the central body -6- and the slotted base plate -7- and allowing to carry out an angular motion between both parts as per the arrow -F'- (see Fig. 4A), said angular motion being lockable at different angular values by means of the incorporation of a tubular structure -27- having an arcuate concave front face and establishing contact with a projection -28a- of the back face -28- of the base plate -7- of the fixing device -5b-, a special spring -29- being provided which maintains the arcuate, tubular structure with the locking notches in position against the base plate body.

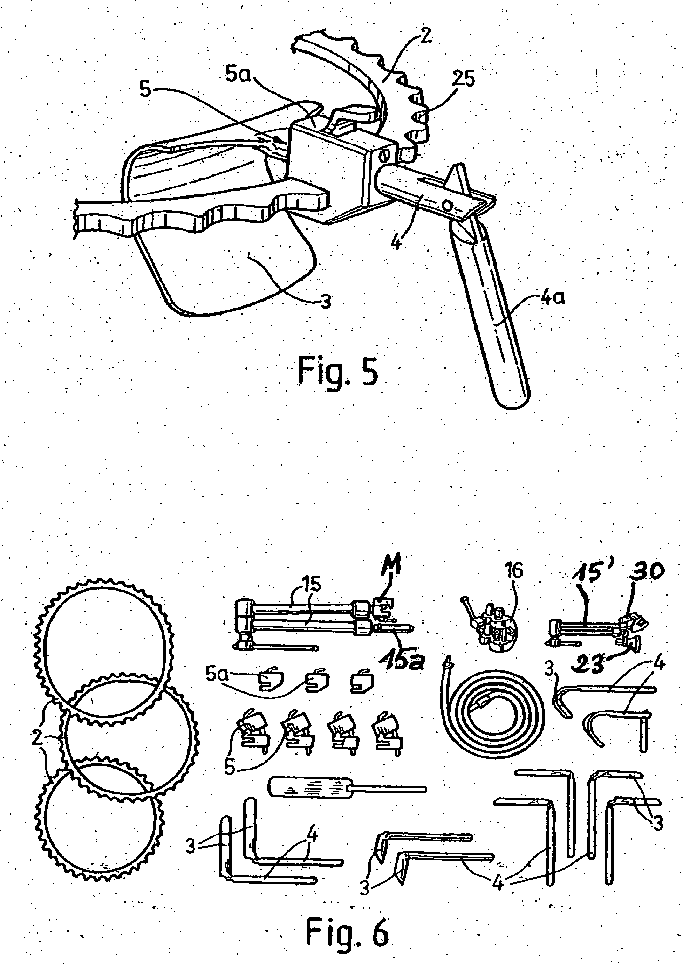

The fixing device of the fixed type -5a- in its turn consists in a one-piece member integrally comprising the central body and the slotted base plate as shown in Fig. 5.

As for the multiarticulated supporting arm -15-, a second tube -17'-, -18'- is apt to slide inside each tube -17-, -18-, said second tube ending in a 45° chamfering at the level of the central locking thread -20-, in such a way that in said locking arrangement the locking thread articulates two bodies having a tapered section such that when threadingly tightening the assembly the taperingly driving bodies are brought nearer to one another thus simultaneously pushing the inner tubes -17'-, -18'- of each tube against the ball joint arrangement being provided at each end, the whole assembly making up the articulated arm -15- being thus totally locked, a three-dimensional movability being thus provided for the ball joint arrangement in combination with the thread. Said, multiarticulated supporting arm -15- is fixed to the ring by means of an articulated clamp arrangement -M- being apt to be tightened from the top by means of the corresponding thumbscrew or the like and to be adapted to any arc, thickness and size of the ring -2- in question (see Fig. 11).

The orientable cold light -23- has a mechanism like that of the supporting arm -15- comprising two tubular structures -15'-, -15'- (Fig. 7) being articulated at one of their ends by means of a central locking thread and at the opposite end of each tube having a ball joint arrangement at one side articulating the rod being incorporated into the arm's lateral support being provided for supporting the lamp, said ball joint arrangement at the other side articulating the gripping clamp -30- being provided for the fixation to the field opening ring -2-, said mechanism being such that a second tube is apt to slide inside each tube, said second tube ending in a 45° chamfering at the level of the central locking thread, in such a way that in said locking arrangement the locking thread articulates two bodies having a tapered section such that when threadingly tightening the assembly the taperingly driving bodies are brought nearer to one another thus simultaneously pushing the inner tubes of each tubular member against the ball joint arrangement being provided at each end, the whole assembly making up the articulated arm being thus totally locked, a three-dimensional movability being thus provided for the ball joint arrangement in combination with the thread.

According to the invention the separator as a whole and its different components and accessories are to be made of a stainless steel based material as befits the sanitary-hygienic maintenance having to be indispensably carried out for this type of instrumentarium.

It shall be understood that when practically embodying the autostatic surgical separator and the different components and accessories of the invention all detail changes as deemed opportune will be acceptable as long as they fall within the scope of the following claims.

## Claims

1. Autostatic surgical separator for automatically and *en bloc* holding and separating the skin and the tissue planes around the lips of the surgical incision, said autostatic surgical separator comprising a field opening ring (2) being provided for opening the field within said lips, pulling valvae (3) being provided with arms for pulling said lips, fixing devices (5) being applied along the field opening ring, supporting means being provided for supporting field the opening ring, and lighting means being provided for lighting the field area; wherein
- said field opening ring (2) is open or closed ;
- said pulling valvae (3) are of different configurations and are integral with the corresponding pulling arm (4) having a smooth surface and any cross-section, this latter being preferentially circular or oval-shaped;
- said fixing devices (5) allowing to fix respective pulling valvae (3) are fitted into them by means of the corresponding arms (4) to the field opening ring, said fixing devices comprising a central body (6) being fitted to a base plate (7) being provided with a slot (8) and by means of which the fixing device is fixed to the field opening ring (2), said central body having a longitudinal channel (9) whose cross-section is in correspondence with that of the pulling arm (4) of the respective valva (3), said central body as well upperly having a transversal slit (10) allowing to fit into it an apertured dog (11) being made up by a plate (11a) being provided with an aperture (11b) whose contour is with a slight play in correspondence with the cross-section of the valva arm (4), said plate being provided with an appendage (11c) making up the dog itself, the valva pulling arm (4) having a cross-section being with a slight play in correspondence with that of said aperture being apt to slide through said aperture (11b) of the dog plate, said upper apertured dog being permanently biased in a forward direction by a stub being in its turn biased by resilient means, said arrangement allowing the pulling arm (4) of the pulling valva (3) to slide through the aperture (11b) of the plate of the upper apertured dog (11) when sliding through the longitudinal channel (9), the aperture of said apertured dog (11) locking the pulling arm (4) of the pulling valva (3) when this latter attempts to move in the same direction and in the opposite sense, the pulling arm (4) thus being locked inside the fixing device (5), the locking pressure being exerted by the apertured dog (11) on the pulling arm (4) becoming higher when there is an increase of the pressure being exerted on the pulling valva (3), said locking being released when the user proceeds to digitally actuate said apertured dog (11) in a backward direction thus allowing the pulling arm (4) of the corresponding pulling valve (3) to be easily slid; said fixing device (5) being of a fixed (5a) or tiltable (5b) type with respect to the field opening ring (2);
- a multiarticulated supporting arm (15) is provided to support the field opening ring (2) and having a mechanism (16) allowing to quickly and easily fix one end (21) of said arm to on exterior corresponding support and the opposite end (22) to the field opening ring (2) by means of an articulated, two-membered clamp arrangement (M), said supporting arm comprising two tubular structures (15, 17, 18) being articulated at one of their ends by a central locking thread (20) and nut (19) and at the other end (21, 22) of each tube having a ball joint arrangement at one side (21) engaging a rod (15a) of the supporting arm to the exterior support (16) and at the other side (22) the clamp (M) being provided to fix the field opening ring (2); and
- said lighting neons comprising an orientable cold light (23) being provided at the end of a multiarticulated arm (15') being similar to the above-mentioned supporting arm and having a mechanism allowing to quickly and easily fix one end of said supporting arm by a gripping clamp (30) to the field opening ring (2) said orientable cold light being provided at the opposite end, said orientable cold light (23) comprising a cup (23a) being provided with a lens (23b) being fitted to one end and a thread (23d) at the opposite end, the multiarticulated arm (15') being fixed (23e) to said thread, the end of an optical fibre cable (23f) being fitted to said opposite end.

2. Autostatic surgical separator according to claim 1, **characterized in that** the field opening ring (2) having an open or closed configuration is essentially planar and is exteriorly provided with a succession of arcuate notches

3. Autostatic surgical separator according to claim 1, **characterized in that** the pulling arms (4) have a smooth surface and any cross-section, this latter preferentially being circular or oval-shaped, the aperture (11b) of the plate of the apertured dog (11) of the fixing device (5) in correspondence with said cross-sectional shape having the same cross-section with a slight play being preferentially existent in the vertical plane.

4. Autostatic surgical separator according to claim 3, **characterized in that** the pulling arms (4) are made up by a rigid one-piece member.

5. Autostatic surgical separator according to 3, **characterized in that** the pulling arms (4) are articulated (4a) at an intermediary position thus occupying less space.

6. Autostatic surgical separator according to claim 1 **characterized in that** the tiltable fixing device (5b) comprises a swivel pin (26) being located at the front face between the central body (6) and the slotted base plate (7) and allowing to carry out an angular motion between both parts, said angular motion being lockable at different angular values by means of the incorporation of a tubular structure (27) having an arcuate concave front face and establishing contact with the back face (28) of the base plate (7) of the fixing device (5), a special spring (29) being provided which maintains the arcuate, tubular structure (27) with the locking notches (27a) in engagement with a projection (28a) of the body (28) of the base plate (7) thereby maintaining the fixing device (5b) in position with the desired inclination.

7. Autostatic surgical separator according to claim 1 **characterized in that** the fixing device of the fixed type (5a) in its turn consists in a one-piece member integrally comprising the central body (6) and the slotted base plate (7).

8. Autostatic surgical separator according to claim 1, **characterized in that** in the mechanism of the multiarticulated supporting arm (15) a second tube (17', 18') is apt to slide inside each tube (17, 18) said second tube ending in a 45° chamfering at the level of the central locking (20, 19) thread, in such a way that in said locking arrangement the locking thread articulates two bodies (20a, 19a) having a tapered section such that when threadingly tightening the assembly the taperingly driving bodies are brought nearer to one another thus simultaneously pushing the inner tubes (17', 18') of each tubular member against the ball joint arrangement being (21, 22) provided at each end, the whole assembly making up the articulated arm (15) being thus totally locked, a three-dimensional movability being thus provided for the ball joint arrangement in combination with the thread (20, 19).

9. Autostatic surgical separator according to claim 1, **characterized in that** the mechanism of the orientable cold light (23) comprises two tubular structures (15', 15') being articulated at one of their ends by means of a central locking thread (20', 19') and at the opposite end of each tube having a ball joint arrangement (21', 22') at one side (21') a rod (23e) being provided for supporting the lamp, said ball joint arrangement at the other side having the gripping clamp (30) being provided for the fixation to the field opening ring, said mechanism being such that a second tube is apt to slide inside each tube (15', 15') said second tube ending in a 45° chamfering at the level of the central locking thread (20', 19') in such a way that in said locking arrangement the locking thread articulates two bodies (20'a, 19'a) having a tapered section such that when threadingly tightening the assembly the taperingly driving bodies are brought nearer to one another thus simultaneously pushing the inner tubes of each tube against the ball joint arrangement (21', 22') being provided at each end, the whole assembly making up the articulated arm being thus totally locked, a three-dimensional movability being thus provided for the ball joint arrangement in combination with the thread (20', 19').

10. Autostatic surgical separator according to the preceding claims, **characterized in that** the valvae arms (4), the aperture (11b) of the plate of the apertured dog (11) and the longitudinal channel (9) of the fixing device (5) preferentially have a circular cross-section/contour allowing to fit the arms into the fixing device (5) in any angular position.

11. Autostatic surgical separator according to the receding claims, **characterized in that** the bottom of the slot (8) of the base plate (7) of the fixing device (5) is flat and hence allows to fit and connect the fixing device (5) at the valleys and peaks of the notches (25) of the ring (2).

## Patentansprüche

1. Autostatischer chirurgischer Retraktor zum selbsttätigen Zurückhalten und Spreizen der Haut und der Gewebeebenen im Ganzen um die Lippen des chirurgischen Einschnitts, wobei der vorgenannte autostatische chirurgische Retraktor einen zum Öffnen des Felds innerhalb der vorerwähnten Lippen bestimmten Feldöffnungsring (2), zum Ziehen der obengenannten Lippen bestimmte, mit Armen versehene Zugvalven (3), entlang des Feldöffnungsrings angebrachte Befestigungsvorrichtungen (5), zum Halten des Feldöffnungsrings bestimmte Haltemittel und zur Beleuchtung des Feldbereichs bestimmte Beleuchtungsmittel umfaßt; worin:
- der vorgenannte Feldöffnungsring (2) als offener oder geschlossener Ring ausgebildet ist;
- die obengenannten Zugvalven (3) verschiedene Ausbildungen haben und mit dem entsprechenden Zugarm (4) einstückig sind, wobei dieser letztere eine glatte Oberfläche und irgendwelchen, vorzugsweise kreisförmigen oder ovalen Querschnitt hat;
- die vorgenannten Befestigungsvorrichtungen (5) es erlauben, die jeweiligen durch die entsprechenden Arme (4) in ihnen eingesetzten Zugvalven (3) zum Feldöffnungsring zu befestigen, wobei die vorerwähnten Befestigungsvorrichtungen einen Zentralkörper (6) umfassen, der mit einer Basisplatte (7) verbunden ist, die mit einer Nut (8) versehen ist und dazu dient, die Befestigungsvorrichtung zum Feldöffnungsring (2) zu befestigen, wobei der vorgenannte Zentralkörper einen Längskanal (9) hat dessen Querschnitt demjenigen des Zugarms (4) der jeweiligen Valve (3) angepaßt ist, wobei der obengenannte Zentralkörper in seinem oberen Bereich auch einen Querschlitz (10) aufweist, der einen durchgebohrten Rastwinkel (11) aufnimmt, der aus einer Platte (11a) besteht, die mit einer Öffnung (11b) versehen ist deren Umfang dem Querschnitt des Valvenarms (4) mit einem geringfügigen Spiel angepaßt ist, wobei die vorgenannte Platte mit einem den eigentlichen Rastwinkel bildenden Absatz (11c) versehen ist, wobei der einen demjenigen der vorgenannten Öffnung mit einem geringfügigen Spiel angepaßten Querschnitt aufweisende Valvenzugarm (4) durch die vorerwähnte Öffnung (11b) der Rastwinkelplatte durchschiebbar ist, wobei der vorerwähnte durchgebohrte Rastwinkel von einem seinerseits von Federmitteln vorgespannten Stift ständig nach vorne vorgespannt wird, wobei die vorgenannte Anordnung es erlaubt, den Zugarm (4) der Zugvalve (3) durch die Öffnung (11b) der Platte des oberen durchgebohrten Rastwinkels (11) während seiner gleitenden Bewegung durch den Längskanal (9) durchzuschieben, wobei die Öffnung des vorgenannten durchgebohrten Rastwinkels (11) eine Einrastung des Zugarms (4) der Zugvalve (3) bei einer versuchten Bewegung dieses letzteren in derselben Richtung und im entgegengesetzten Sinn verursacht, wobei der Zugarm (4) dann auf diese Weise im Inneren der Befestigungsvorrichtung (5) verriegelt wird, wobei der vom durchgebohrten Rastwinkel (11) auf den Zugarm (4) ausgeübte Verriegelungsdruck bei einer Erhöhung des auf die Zugvalve (3) ausgeübten Drucks sich erhöht, wobei die vorgenannte Verriegelung bei einer anwenderseitigen Fingerbetätigung des obenerwähnten durchgebohrten Rastwinkels (11) nach hinten aufgehoben wird und **dadurch** eine leichte Durchschiebbarkeit des Zugarms (4) der entsprechenden Zugvalve (3) ermöglicht, wobei die vorgenannte Befestigungsvorrichtung (5) eine in bezug auf den Feldöffnungsring (2) starre (5a) oder schwenkbare (5b) Bauart aufweist;
- ein mehrgelenkiger Haltearm (15) dazu vorgesehen ist, den Feldöffnungsring (2) zu halten und einen Haltemechanismus hat, der es erlaubt, ein Ende (21) des vorgenannten Arms zu einem entsprechenden äußeren Halter (16) und das entgegengestzte Ende (22) durch eine gelenkige, zweiteilige Klemmbackenanordnung (M) zum Feldöffnungsring (2) zu befestigen, wobei der vorgenannte Haltearm zwei rohrförmige Strukturen (15, 17, 18) umfaßt, die an einem ihrer Enden durch ein Zentralverriegelungsgewinde (20) und eine Gewindemutter (19) gelenkig gemacht werden und am anderen Ende (21, 22) jedes Rohrs eine Kniegelenkanordnung haben, die an einer Seite (21) den Zapfen (15a) des Haltearms mit dem äußeren Halter (16) verbindet und an der anderen Seite (22) mit der Klemmbackenanordnung (M) zur Befestigung zum Feldöffnungsring (2) versehen ist; und
- eine orientierbare Kaltleuchte (23) am Ende eines mehrgelenkigen Arms (15') vorgesehen ist, wobei dieser letztere dem vorgenannten Haltearm ähnelt und einen Mechanismus hat, der es erlaubt, ein Ende des vorerwähnten Haltearms mittels einer Klemmbacke (30) zum Feldöffnungsring (2) in schneller und einfacher Weise zu befestigen, wobei die vorgenannte orientierbare Kaltleuchte am entgegengesetzten Ende vorgesehen ist, wobei die vorerwähnte Kaltleuchte (23) eine Pfanne (23a) umfaßt, die mit einer mit einem Ende verbundenen Linse (23b) und einem Gewinde (23d) am gegenüberliegenden Ende versehen ist, wobei der mehrgelenkige Arm (15') zum vorgenannten Gewinde befgestigt (23e) ist, wobei das Ende eines Lichtleitfaserkabels (23f) mit den vorerwähnten gegenüberliegenden Ende verbunden ist.

2. Autostatischer chirurgischer Retraktor nach Anspruch 1, **dadurch gekennzeichnet, daß** der eine offene oder geschlossene Ausbildung aufweisende Feldöffnungsring (2) im wesentlichen planar und außenseitig mit einer Reihe aufeinenderfolgender, bogenförmiger Einkerbungen versehen ist.

3. Autostatischer chirurgischer Retraktor nach Anspruch 1, **dadurch gekennzeichnet, daß** die Zugarme (4) eine glatte Oberfläche und irgendwelchen, vorzugsweise kreisförmigen oder ovalen Querschnitt haben, wobei die dem vorgenannten Querschnitt angepaßte Öffnung (11b) der Platte des durchgebohrten Rastwinkels (11) der Befestigungsvorrichtung (5) denselben Querschnitt mit einem geringfügigen, vorzugsweise in der Senkrechtebene bestehenden Spiel hat.

4. Autostatischer chirurgischer Retraktor nach Anspruch 3, **dadurch gekennzeichnet, daß** die Zugarme (4) aus einem starren, einstückigen Teil bestehen.

5. Autostatischer chirurgischer Retraktor nach Anspruch 3, **dadurch gekennzeichnet, daß** die Zugarme (4) an einem Mittelpunkt gelenkig gemacht sind und **dadurch** einen kleineren Raumbedarf haben.

6. Autostatischer chirurgischer Retraktor nach Anspruch 1, **dadurch gekennzeichnet, daß** die schwenkbare Befestigungsvorrichtung (5b) eine Schwenkachse (26) umfaßt, die sich an der Vorderseite zwischen dem Zentralkörper (6) und der genuteten Basisplatte (7) befindet und die Durchführung einer Winkelbewegung zwischen beiden Teilen ermöglicht, wobei die vorgenannte Winkelbewegung in verschiedenen Winkelstellungen durch die Eingliederung einer eine konkave, bogenförmige Vorderseite aufweisenden, mit der Hinterseite (28) der Basisplatte (7) der Befestigungsvorrichtung (5) in Berührung kommenden, rohrförmigen Struktur (27) arretierbar ist, wobei eine Sonderfeder (29) vorgesehen ist, die die rohrförmige Struktur (27) mit den Rastkerben (27a) in Eingriff mit einem Vorsprung (28a) des Körpers (28) der Basisplatte (7) hält und **dadurch** die Befestigungsvorrichtung (5b) in Position mit der gewünschten Neigung hält.

7. Autostatischer chirurgischer Retraktor nach Anspruch 1, **dadurch gekennzeichnet, daß** die Befestigungsvorrichtung der starren Bauart (5a) ihrerseits aus einem einstückigen, den Zentralkörper (6) und die genutete Basisplatte (7) umfassenden Teil besteht.

8. Autostatischer chirurgischer Retraktor nach Anspruch 1, **dadurch gekennzeichnet, daß** im Mechanismus des mehrgelenkigen Haltearms (15) ein zeites Rohr (17', 18') in gelitender Anordnung innerhalb des jeweiligen Rohrs (17, 18) angeordnet ist, wobei das vorgenannte zeite Rohr in einer 45°-igen Abfasung im Bereich des Zentralverriegelungsgewindes (20, 19) endet, auf solche Weise, daß in der vorgenannten Verriegelungsanordnung das Verriegelungsgewinde zwei sich konisch verjungende Körper (20a, 19a) auf solche Weise betätigt, daß beim Gewindeanziehen der gesamten Anordnung die konisch betätigende Körper gegenseitig angenähert werden und so gleichzeitig die inneren Rohre (17', 18') jedes rohrförmigen Teils gegen die an jeden Ende vorgesehene Kniegelenkanordnung (21, 22) schiebt, wobei die Gesamtanordnung die den mehrgelenkigen Haltearm (15) bildet auf diese Weise ganz verriegelbar ist, wobei eine dreidimensionale Bewegbarkeit für die Kniegelenkanordnung in Verbindung mit dem Gewinde (20, 19) geschafft wird.

9. Autostatischer chirurgischer Retraktor nach Anspruch 1, **dadurch gekennzeichnet, daß** der Mechanismus der orientierbaren Kaltleuchte (23) zwei rohrförmige Strukturen (15', 15') umfaßt, die an einem ihrer Enden durch ein Zentralverriegelungsgewinde (20', 19') gelenkig gemacht sind und am gegenüberliegenden Ende jedes Rohrs eine Kniegelenkanordnung (21', 22') haben, wobei an einer Seite (21') ein Zapfen (23e) zum Halten der Leuchte vorgesehen ist, wobei die vorgenannte Kniegelenkanordnung an der anderen Seite die zur Befestigung zum Feldöffnungsring vorgesehene Klammbacke (30) hat, wobei der vorerwähnte Mechanismus so angeordnet ist, daß ein zweites Rohr in gleitender Anordnung innerhalb des jeweiligen Rohrs (15', 15') angeordnet ist, wobei das vorgenannte zeite Rohr in einer 45°-igen Abfasung im Bereich des Zentralverriegelungsgewindes (20', 19') endet, auf solche Weise, daß in der vorgenannten Verriegelungsanordnung das Verriegelungsgewinde zwei sich konisch verjungende Körper (20'a, 19'a) auf solche Weise betätigt, daß beim Gewindeanziehen der gesamten Anordnung die konisch betätigende Körper gegenseitig angenähert werden und so gleichzeitig die inneren Rohre jedes Rohrs gegen die an jeden Ende vorgesehene Kniegelenkanordnung (21', 22') schiebt, wobei die Gesamtanordnung die den mehrgelenkigen Haltearm bildet auf diese Weise ganz verriegelbar ist, wobei eine dreidimensionale Bewegbarkeit für die Kniegelenkanordnung in Verbindung mit dem Gewinde (20', 19') geschafft wird.

10. Autostatischer chirurgischer Retraktor nach den vorhergehenden Ansprüchen, **dadurch gekannzeichnet, daß** die Valvenarme (4), die Öffnung (11b) der Platte des durchgebohrten Rastwinkels (11) und der Längskanal (9) der Befestigungsvorrichtung (5) vorzugsweise einen kreisförmigen Querschnitt/Umfang haben, der es erlaubt, die Arme in der Befestigungsvorrichtung in irgendwelcher Winkellage aufzunehmen.

11. Autostatischer chirurgischer Retraktor nach den vorhergehenden Ansprüchen, **dadurch gekannzeichnet, daß** der Grund der Nut (8) der Basisplatte (7) der Befestigungsvorrichtung (5) flach ist und deswegen es erlaubt, die Befestigungsvorrichtung (5) an den Spitzen und Tälern der Einkerbungen (25) des Rings(2) zu stecken und zu verbinden.

## Revendications

1. Écarteur chirurgical autostatique pour retenir et écarter automatiquement en bloc la peau et les plans tissulaires autour des lèvres de l'incision chirurgicale, ledit écarteur chirurgical autostatique comprenant un anneau (2) d'ouverture de champ étant prévu pour l'ouverture du champ entre lesdites lèvres, des valves de traction (3) munies de bras pour tirer lesdites lèvres, des dispositifs de fixation (5) appliqués au long de l'anneau d'ouverture de champ, des moyens de support prévus pour supporter l'anneau d'ouverture de champ, et des moyens d'éclairage prévus pour éclairer la zone de champ; où:
- ledit anneau (2) d'ouverture de champ est ouvert ou fermé;
- lesdites valves de traction (3) sont de différentes configurations et sont venues de matière avec le correspondant bras (4) de traction ayant une surface lisse et n'importe quelle section transversale, celle-ci étant préférentiellement circulaire ou ovale;
- lesdits dispositifs de fixation (5) permettent de fixer des respectives valves de traction (3) à l'anneau d'ouverture de champ au moyen des correspondants bras (4), lesdits dispositifs de fixation comprenant un corps central (6) étant accouplé à une plaque de base (7) munie d'une rainure (8) au moyen de laquelle le dispositif de fixation est fixé à l'anneau (2) d'ouverture de champ, ledit corps central ayant un canal longitudinal (9) à section transversale en correspondance avec celle du bras (4) de traction de la correspondante valve (3), ledit corps central présentant aussi, dans sa partie supérieure, une fente (10) transversale permettant d'y accoupler une patte perforée (11) constituée d'une plaque (11a) munie d'une ouverture (11b) dont le contour est avec un léger jeu en correspondance avec la section transversale du bras (4) de la valve, ladite plaque étant munie d'un appendice (11c) constitutif de la patte proprement dite, le bras (4) de traction de la valve ayant une section transversale qui avec un léger jeu est en correspondance avec celle de ladite ouverture et étant apte à glisser à travers ladite ouverture (11b) de la plaque de la patte, ladite patte perforée supérieure étant d'une manière permanente biaisée vers l'avant par un téton étant à son tour biaisé par des moyens élastiques, ladite disposition permettant au bras (4) de traction de la valve (3) de traction de glisser à travers l'ouverture (11b) de la plaque de la patte perforée (11) supérieure lors de son glissement à travers le canal longitudinal (9), l'ouverture de ladite patte perforée (11) bloquant le bras (4) de traction de la valve (3) de traction lorsque celui-là essaye de se déplacer dans la même direction et en sens opposé, le bras (4) de traction restant ainsi bloqué dans le dispositif de fixation (5), la pression de blocage exercée par la patte perforée (11) sur le bras (4) de traction augmentant lorsque la pression exercée sur la valve (3) de traction s'accroît, ledit blocage se relâchant lorsque l'utilisateur actionne avec le doigt ladite patte perforée (11) vers l'arrière, permettant ainsi au bras (4) de traction de la valve (3) de traction correspondante de glisser facilement; ledit dispositif de fixation (5) étant du type fixe (5a) ou à bascule (5b) par rapport à l'anneau (2) d'ouverture de champ;
- un bras de support (15) multi-articulé est prévu pour supporter l'anneau (2) d'ouverture de champ et a un mécanisme (16) permettant la fixation rapide et facile d'une extrémité (21) dudit bras à un support extérieur correspondant et celle de l'extrémité opposée (22) à l'anneau (2) d'ouverture de champ au moyen d'un système de fixation à double mâchoire (M) articulé, ledit bras de support comprenant deux structures tubulaires (15, 17, 18) articulées à l'endroit de l'une de leurs extrémités par un filet de blocage (20) central et un écrou (19) et ayant à l'endroit de l'autre extrémité (21, 22) de chaque tube un système à rotule (21) engageant sur un côté une tige (15a) du bras de support avec le support extérieur (16) et sur l'autre la mâchoire (M) de fixation de l'anneau (2) d'ouverture de champ; et
- lesdits moyens d'éclairage comprennent une lumière froide (23) orientable étant prévue à l'extrémité d'un bras (15') multi-articulé similaire au bras de support susmentionné et présentent un mécanisme permettant une fixation rapide et facile, au moyen d'une mâchoire (30) de préhension, d'une extrémité dudit bras de support à l'anneau (2) d'ouverture de champ, ladite lumière orientable froide étant prévue à l'extrémité opposée, ladite lumière orientable froide (23) comprenant une cassolette (23a) munie d'une lentille (23b) étant accouplée à une extrémité et d'un filet (23d) à l'extrémité opposée, le bras (15') multi-articulé étant fixé (23e) audit filet, l'extrémité d'un câble fibre optique (23f) étant accouplée à ladite extrémité opposée.

2. Écarteur chirurgical autostatique, selon la revendication 1, **caractérisé en ce que** l'anneau (2) d'ouverture de champ à configuration ouverte ou fermée est essentiellement planaire et est extérieurement muni d'une succession d'encoches arquées (25).

3. Écarteur chirurgical autostatique, selon la revendication 1, **caractérisé en ce que** les bras (4) de traction ont une surface lisse et n'importe quelle section transversale, celle-ci étant préferentiellement circulaire ou ovale, l'ouverture (11b) de la plaque de la patte perforée (11) du dispositif de fixation (5) en correspondance avec ladite section transversale ayant la même section transversale avec un léger jeu étant préférentiellement présent dans le plan vertical.

4. Écarteur chirurgical autostatique, selon la revendication 3, **caractérisé en ce que** les bras (4) de traction sont constitués d'un élément monopièce rigide.

5. Écarteur chirurgical autostatique, selon la revendication 3, **caractérisé en ce que** les bras (4) de traction sont articulés (4a) dans une position intermédiaire, prenant ainsi moins d'espace.

6. Écarteur chirurgical autostatique, selon la revendication 3, **caractérisé en ce que** le dispositif de fixation à bascule (5b) comprend un axe de pivotement (26) situé sur la face frontale entre le corps central (6) et la plaque de base rainurée (7) et permettant d'effectuer un mouvement angulaire entre les deux pièces, ledit mouvement angulaire étant blocable à différentes valeurs angulaires au moyen de l'incorporation d'une structure tubulaire (27) ayant une face avant concave et arquée et rentrant en contact avec la face arrière (28) de la plaque de base (7) du dispositif de fixation (5), un ressort spécial (29) étant prévu qui maintient la structure tubulaire arquée (27) avec les rainures de blocage (27a) en engagement avec une saillie (28a) du corps (28) de la plaque de base (7), maitenant ainsi le dispositif de fixation (5b) en position avec l'inclinaison souhaitée.

7. Écarteur chirurgical autostatique, selon la revendication 1, **caractérisé en ce que** le dispositif de fixation du type fixe (5a) consiste à son tour en un élément monopièce comprenant intégralement le corps central (6) et la plaque de base rainurée (7).

8. Écarteur chirurgical autostatique, selon la revendication 1, **caractérisé en ce que** dans le mécanisme du bras de support (15) multi-articulé un second tube (17', 18') est apte à glisser à l'intérieur de chaque tube (17, 18), ledit second tube finissant par un biseau de 45° au niveau du filet de blocage central (20, 19), de façon à ce que dans ledit système de blocage le filet de blocage articule deux corps (20a, 19a) à section conique de sorte que lors du serrage à vis de l'ensemble les corps à traction conique se rapprochent l'un de l'autre, poussant ainsi en même temps les tubes intérieurs (17', 18') de chaque élément tubulaire contre le système à rotule (21, 22) étant prévu à chaque extrémité, tout cet ensemble constitutif du bras articulé (15) restant ainsi totalement bloqué, une mobilité tridimensionelle étant ainsi fournie au système à rotule en combinaison avec le filet (20, 19).

9. Écarteur chirurgical autostatique, selon la revendication 1, **caractérisé en ce que** le mécanisme de la lumière froide orientable (23) comprend deux structures tubulaires (15', 15') articulées à l'endroit de l'une de leurs extrémités au moyen d'un filet de blocage central (20', 19') et ayant, à l'extrémité opposée de chaque tube, un système à rotule (21', 22') ayant sur un côté (21') une tige (23e) étant prévue pour supporter la lampe, ledit système à rotule ayant sur l'autre côté la mâchoire de préhension (30) étant prévue pour la fixation de l'anneau (2) d'ouverture de champ, ledit mécanisme étant tel qu'un second tube est apte à glisser dans chaque tube (15', 15'), ledit second tube finissant par un biseau de 45° au niveau du filet de blocage central (20', 19'), de façon à ce que dans ledit système de blocage le filet de blocage articule deux corps (20'a, 19'a) à section conique de sorte que lors du serrage à vis de l'ensemble les corps à traction conique se rapprochent l'un de l'autre, poussant ainsi en même temps les tubes intérieurs de chaque tube contre le système à rotule (21', 22') étant prévu à chaque extrémité, tout cet ensemble constitutif du bras articulé restant ainsi totalement bloqué, une mobilité tridimensionelle étant ainsi fournie au système à rotule en combinaison avec le filet (20', 19').

10. Écarteur chirurgical autostatique, selon les revendications précédentes, **caractérisé en ce que** les bras (4) des valves, l'ouverture (11b) de la plaque de la patte perforée (11) et le canal longitudinal (9) du dispositif de fixation (5) présentent préférentiellement une section transversale/contour permettant d'accoupler les bras dans le dispositif de fixation (5) dans n'importe quelle position angulaire.

11. Écarteur chirurgical autostatique, selon les revendications précédentes, **caractérisé en ce que** le fond de la rainure (8) de la plaque de base (7) du dispositif de fixation (5) est plat, permettant ainsi d'accoupler et connecter le dispositif de fixation (5) dans les vallées et les sommets des encoches (25) de l'anneau (2).
